# EUROPEAN PATENT SPECIFICATION

(11) **EP 3 388 100 B1**
(45) Date of publication and mention of the grant of the patent: **18.03.2020**
(21) Application number: 17165991.5
(22) Date of filing: 11.04.2017
(51) Int. Cl.: A61M 16/04, A61M 25/10

(54) **CUFF PRESSURE REGULATOR FOR ENDOTRACHEAL TUBES**
MANSCHETTENDRUCKREGLER FÜR ENDOTRACHEALRÖHREN
RÉGULATEUR DE PRESSION DE BRASSARD DESTINÉ À DES TUBES ENDOTRACHÉAUX

(43) Date of publication of application: 17.10.2018
(73) Proprietor: Asset Medikal Tasarim Sanayi ve Ticaret A.S., Basaksehir-Istanbul (TR)
(72) Inventor: TÜYSÜZ, Mehmet, Istanbul (TR); BASARAN, Reha, Istanbul (TR)
(74) Representative: Sevinç, Erkan

(56) References cited:
- WO-A1-98/25663
- WO-A1-2011/133537
- US-A- 4 159 722
- US-A- 4 501 273

## Description

### Technical Field of the Invention

The present invention relates to a device for regulating fluid pressure inside cuffs of endotracheal tubes.

### Background of the Invention

An endotracheal tube generally comprises a cuff to be inflated to seal against the tracheal wall. Insufficient cuff pressures (e.g. lower than about 15-20 mbar) result in the cuff to fail circumferentially abutting to the tracheal wall and thus sealing is compromised. Very high cuff pressures (e.g. higher than about 30-35 mbar) can cause discomfort and even damage around the trachea. Therefore the cuff pressure must be kept within acceptable limits.

The cuff pressure is generally checked by palpating a pilot balloon which is in fluid transfer communication with the cuff. The palpation easily results in subjective evaluation of cuff pressure.

In case where the cuff pressure is outside the acceptable limits, the pressure inside the cuff is manually changed by a user, by manually transferring fluid (e.g. air) into or from the cuff. This results in a high work load to the user(s), and corresponds to a high probability of failing to observe the cuff pressure in time, which may cause severe results such as tracheal damages.

### Objects of the Invention

Primary object of the present invention is to overcome the abovementioned shortcomings of the prior art.

Another object of the present invention is obtainment of a device for automatic regulation of cuff pressure.

Yet another object of the present invention is to provide a low cost and easy to use device for automatic regulation of cuff pressure.

A further object of the present invention is to provide an adjustable device for different cuff pressure ranges.

US 4 159 722 A, WO 2011/133537 A1 and WO 98/25663 A1 relate to devices for regulating cuff pressure in endotracheal tubes. Each of the devices disclosed in said documents necessitate additional syringes for provision of compressed air. Said additional syringes require to be disengaged from said devices for being re-filled, which is to be followed by re-engaging the syringes with the devices. As a result, the use of said devices is highly open to user errors.

### Summary of the invention

The present invention proposes a device for regulating cuff pressure in endotracheal tubes, comprising a barrel for guiding a plunger, the plunger being adapted to travel along a main longitudinal axis of said barrel, thereby defining a cavity with variable volume within the barrel, the device further comprises a passage adapted to allow fluid transfer communication to and from the cavity, and one or more biasing member adapted to bias the plunger towards said passage. The invention is defined in the appended claims.

### Brief Description of the Figure

The figure, whose brief explanation is herewith provided, is solely intended for providing a better understanding of the present invention and is as such not intended to define the scope of protection or the context in which said scope is to be interpreted in the absence of the description.

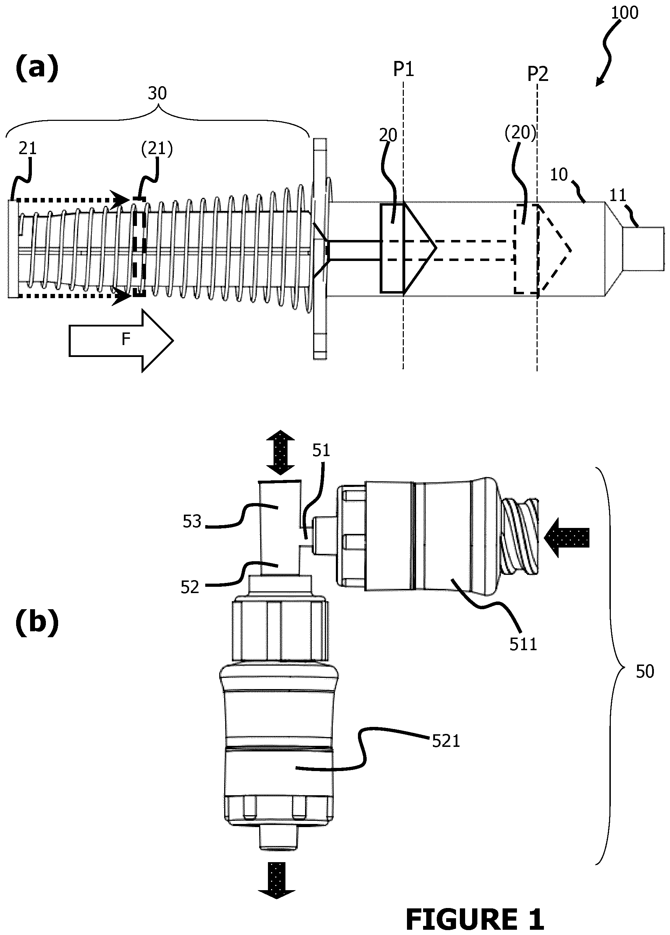

Figure 1 shows (a) a schematic side view of an exemplary embodiment of the device according to the present invention, wherein different positions of the plunger are emphasized; and (b) a tee-connection suitable for coupling with the device.

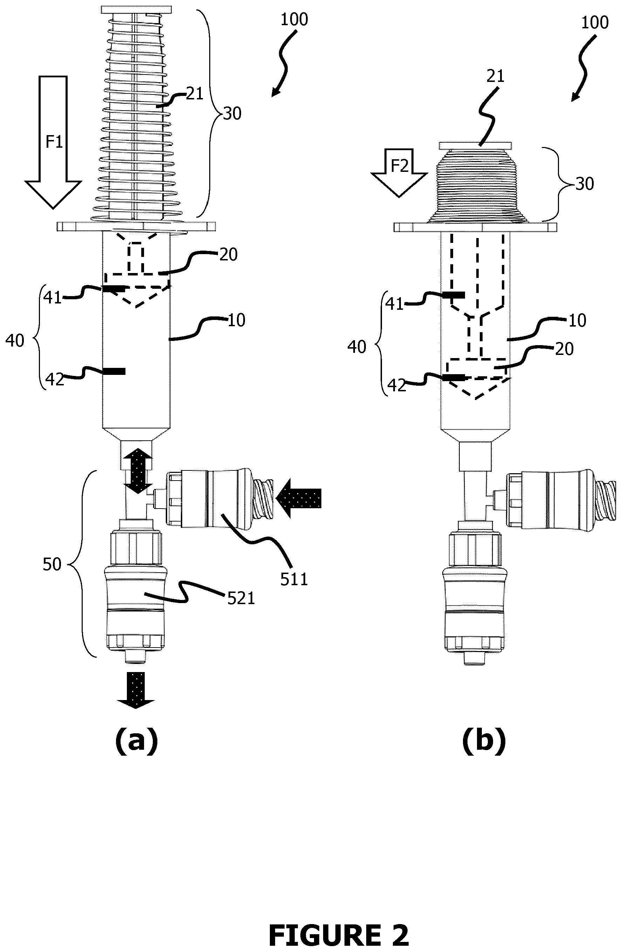

Figure 2 schematically exemplifies an embodiment of the device according to the present invention, provided with the tee-connection, and wherein the biasing member comprises a single resilient member (e.g. a spring with a certain spring constant) (a) at a high pressure state wherein the force of the biasing member is shown as F1, and (b) at a low pressure state wherein the force of the biasing member is shown as F2, which is smaller than F1.

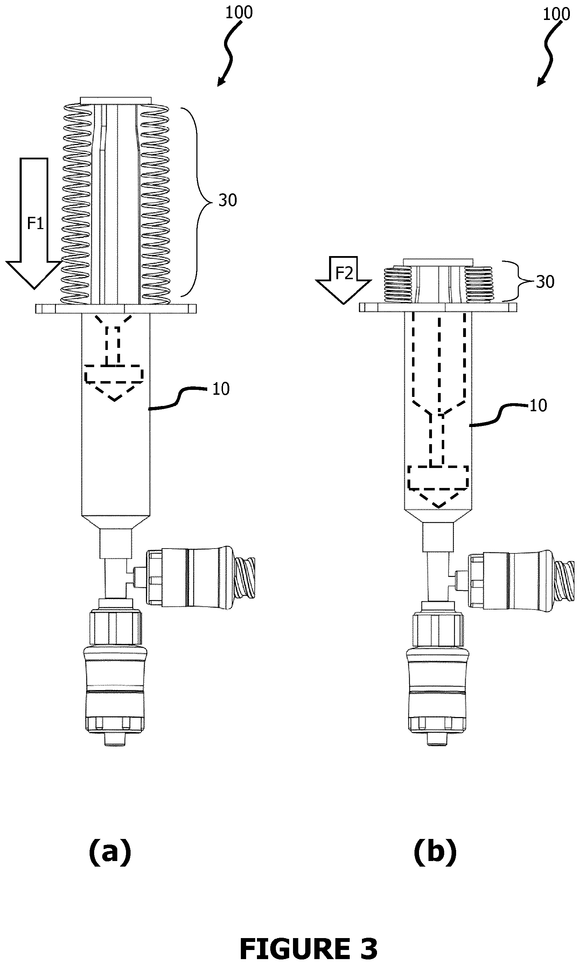

Figure 3 schematically exemplifies another embodiment of the device according to the present invention wherein the biasing member comprises multiple resilient members, (a) at a high pressure state wherein the force of the biasing member is shown as F1, and (b) at a low pressure state wherein the force of the biasing member is shown as F2, which is smaller than F1.

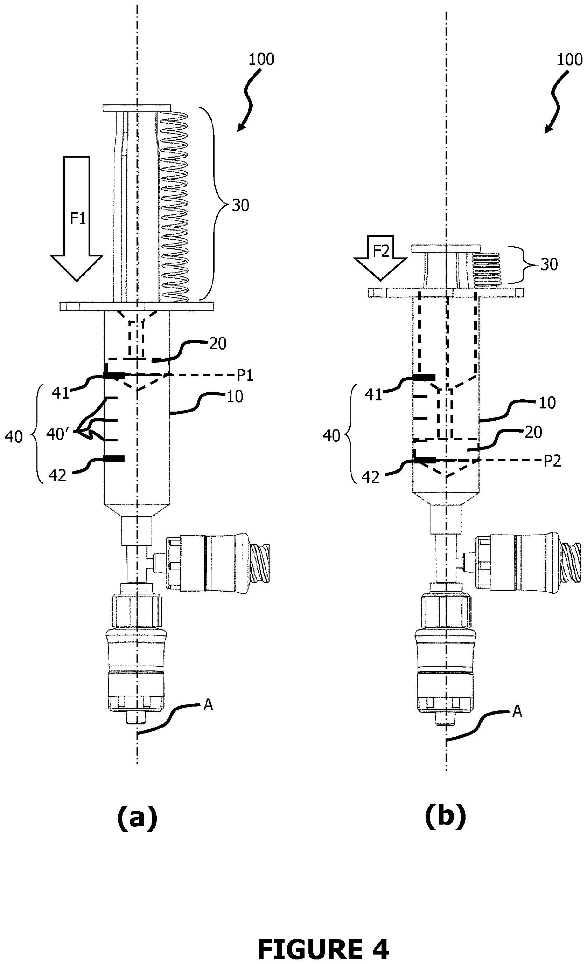

Figure 4 schematically exemplifies another embodiment of the device according to the present invention wherein the biasing member comprises a single resilient member (e.g. the rest of the multiple resilient member(s) being removed for obtaining a lowered pressure range inside the cavity and thus also inside a cuff), (a) at a high pressure state wherein the force of the biasing member is shown as F1, and (b) at a low pressure state wherein the force of the biasing member is shown as F2, which is smaller than F1.

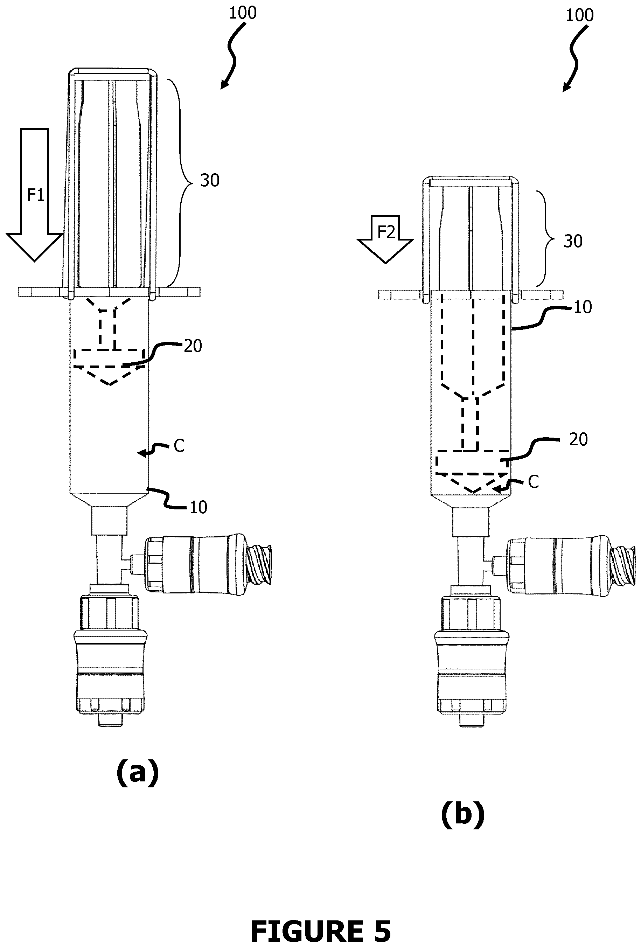

Figure 5 schematically exemplifies another embodiment of the device according to the present invention wherein the biasing member comprises a polymeric elastic member, (a) at a high pressure state wherein the force of the biasing member is shown as F1, and (b) at a low pressure state wherein the force of the biasing member is shown as F2, which is smaller than F1.

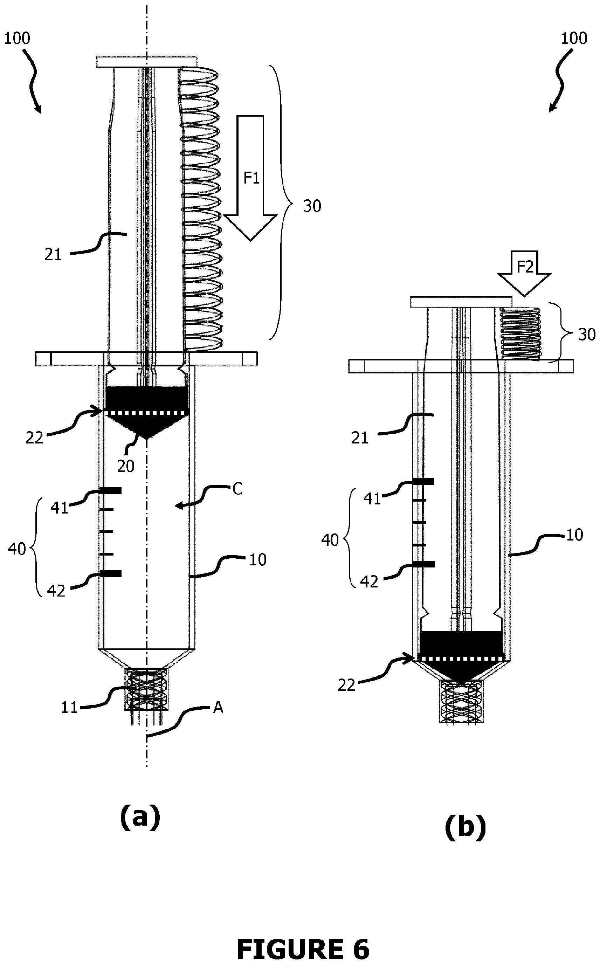

Figure 6 schematically shows an embodiment of the device according to the present invention, adapted to increased precision in visual monitoring by provision of a pressure indicating member on the plunger and a graduated marking scale, at a high pressure state wherein the force of the biasing member is shown as F1, and (b) at a low pressure state wherein the force of the biasing member is shown as F2, which is smaller than F1.

### Detailed Description of the Invention

Referring now the figure outlined before, the present invention proposes a device (100) for regulating cuff pressure in endotracheal tubes. The device (100) comprises a barrel (10) for guiding a plunger (20). Said plunger (20) is adapted to travel along a main longitudinal axis (A) of the barrel (10), thereby defining a cavity (C) with variable volume within the barrel. The device (100) further comprises a passage (11) adapted to allow fluid transfer communication to and from the cavity (C), and one or more biasing member(s) (30) adapted to bias the plunger (20) towards said passage (11).

As the pressure inside a cuff coupled with the device (100) decreases due to leakage of fluid (e.g. air) from the cuff, the pressure decline is at least partly compensated by the device (100), automatically. A mainly closed fluid chamber can be constituted mainly from the cavity (C), the cuff, and any tubing providing fluid transfer communication between the cavity and the cuff. The size (volume) of the cavity is dependent on the distance between the plunger (20) and the passage (11). In the device (100) according to the present invention, higher distances between the plunger and the passage corresponds to higher values of force exerted by the biasing member (30) (e.g. onto the plunger 20, or onto a shaft (21) of the plunger, e.g. in accordance with Hooke's Law).

The distance between the plunger and the passage geometrically corresponds to a respective amount of fluid (e.g. air) contained inside the cavity. As the fluid is gradually fed to the cuff through the passage (11), the volume of the cavity accordingly decreases along with the distance between the plunger (20) and the passage (11). Simultaneously, the force (and the pressure within the cavity, which is mainly equal to that inside the cuff) also drops gradually (e.g. in accordance with Hooke's Law).

Hence, the distance between the plunger (20) and the passage (11) is correlated with the pressure available inside the cavity and also with that inside a cuff coupled with the device (100).

Therefore, the device (100) according to the present invention can enable tracking of the fluid pressure in a cuff (cuff pressure) by visual observation of said distance or of an indicator related to said distance.

The position of the plunger (20) relative to the passage (11) can be observed in many ways including by employing an at least partly transparent barrel (10). For an increased accuracy, a pressure indicating member (22) can be secured to the plunger (20). The pressure indicating member can provide a high contrast relative to the plunger shaft (21), cavity (C) or plunger head by being provided with a color different than at least one of those.

Accordingly, in an embodiment of the present invention, the device (100) further comprises one or more visual indicator(s) (40) (e.g. as exemplified in Fig.2) adapted to allow visual monitoring of a magnitude related to the relative position of the plunger (20) with respect to the passage (11).

Fluid (e.g. air) at a pressure in accordance with the force exerted by the biasing member (30), can automatically transferred into a cuff (of an endortacheal tube) coupled with the device (100). This provides an automatized pressure and volume correction to the fluid inside the cuff. Inacceptable values of cuff pressure (e.g. inacceptably low pressures where the cuff can not function properly) can be visually observed through the visual indicator(s) (40) (e.g. by observing the alignment between the plunger/ or the pressure indicating member (23) and the visual indicator(s) (40)). This eliminates the requirement of any pilot balloon coupled with the endotracheal tube, which is used in palpative examination of cuff pressure causing subjective observations.

In an embodiment of the device (100), the plunger and the barrel can be arranged to define a limited range of motion (e.g. between two positions: P1 and P2) to the plunger (20). In such case, the range of motion can be in accordance with a first value of force (F1) and a second value of force (F1) exerted onto the plunger (20) by the biasing member (30). Said first value (F1) and second value (F2) of force can respectively correspond to a minimum pressure value and a maximum pressure value acceptable inside a cuff of an endotracheal tube, for being functionally halted in a trachea of a patient, but also for avoiding damage related to unacceptable pressure values on the tissues of a patient or discomfort.

The visual indicator (40) can comprise a graduated marking scale (40') (e.g. as exemplified in Fig.4) at a projection mainly parallel to the axis (A), in combination with a position indicating member (22) secured to the plunger (20), which is adapted to enable visual monitoring of the position (P) of the position indicating member (22) relative to said scale (40'). In this embodiment, the scale (40') can be provided with at least a first marker (41), and a second marker (42) which is at a distance different than that of the first marker (41) relative to the passage (11).

The device (100) can be provided in a sleeve (not shown) at least partly covering the device (100). The sleeve can be adapted to allow manual positioning of the plunger inside the barrel (10), but also to allow visual monitoring of the visual indicator (40) from outside of the sleeve.

In such embodiment, the sleeve can be provided with one or more slit (not shown) adapted to allow the position indicating member (22) to protrude through. Preferably, the sleeve can be mainly opaque and the scale (40') can be provided on an outer surface of the sleeve.

In an embodiment according to the present invention, the passage (11) can be provided with a tee-connection (50). The tee-connection (50) can comprise a first conduit (51), a second conduit (52) and a third conduit (53) connected together at one end of each conduit (51, 52 and 53). The first conduit (51) can comprise a first check valve (511) adapted to allow one-way fluid transfer towards the cavity (C), the second conduit (52) can comprise a second check valve (512) adapted to allow one-way fluid transfer from the cavity (C), and the third conduit (53) can be adapted to provide fluid transfer communication between the cavity (C) and both of the first conduit (51) and the second conduit (52).

The distance between the plunger and the passage can be increased manually, thereby pulling fluid (e.g. air) into the cavity, and simultaneously loading potential energy to the biasing member (30) to enable automatic compensation of the cuff pressure (or fluid content inside the cuff).

The biasing member (30) can comprise a resilient member which comprises one or more compression spring adapted to push the plunger (20) towards the passage (11) (not shown). This embodiment is also applicable in case where the device (100) is provided with said sleeve, and the compression spring(s) can be arranged to push the plunger (20) relative to a point of support located on the sleeve.

Alternatively, the biasing member (30) can comprise a resilient member which comprises a tension spring (exemplified in Fig.1a, Fig.2, Fig.4 and Fig.6) or a plurality of tension springs (exemplified in Fig.3) and/or one or more elongate elastic polymeric member (exemplified in Fig.5) adapted to pull the plunger (20) towards the passage (11).

A maximum value (e.g. about 30 mbar) and a minimum value (e.g. about 20 mbar) of acceptable cuff pressures can be selected omitting the different patient needs; and the biasing member can be arranged accordingly (e.g. a universal biasing member for instance with a single elastic member such as a spring with a certain spring constant or an elastic polymeric member with a certain cross-linking ratio) for minimization of costs related to planning-, production- and inventory- related costs of the device (100).

The biasing member (30) can comprise a resilient member which comprises one or more compression spring (not shown) adapted to push the plunger (20) towards the passage (11); and also a further resilient member which comprises one or more tension spring and/or one or more elastic polymeric member adapted to pull the plunger (20) towards the passage (11).

The embodiment where the biasing member (30) comprises multiple elastic members (such as spring(s) and/or elastic polymeric member(s)), enables tailoring of the amount of force exerted by the biasing member to the fluid inside the cavity (which also corresponds to the fluid pressure in a cuff of an endotracheal tube when coupled to the device according to the present invention), regarding to necessities which can be different from case to case. Maximum and minimum values of acceptable cuff pressures can change from patient to patient

The biasing member (30) and the scale (40') can be arranged so that a maximum pressure value acceptable inside a cuff is indicated by the first marker (41) corresponding to the first amount of force to be exerted (e.g. onto the plunger such that, in use, a first value of pressure related to the first amount of force is obtained inside the chamber and a cuff coupled with the device) by the biasing member (30) in case where a projection on the pressure indicating member (22) mainly perpendicular to the axis (A) coincides with the first marker (41), and a minimum pressure value acceptable inside a cuff is indicated by the second marker (42) corresponding to the second amount of force to be exerted (e.g. onto the plunger such that, in use, a second value of pressure related to the second amount of force, which is lower than the first value of pressure, is obtained inside the chamber and a cuff coupled with the device) by the biasing member (30) in case where the projection of the pressure indicating member (22) mainly perpendicular to said axis (A) coincides with the second marker (42).

The present invention accordingly further proposes a kit including an endotracheal tube with a cuff and a device (100) as described above, adapted to couple with the cuff. As an alternative, the present invention further proposes an endotracheal tube comprising a cuff and a device (100) as described above, adapted to couple with the cuff.

Thus the following objects are achieved by the present invention:
- overcoming the abovementioned shortcomings of the prior art,
- provision of:
   ∘ a device for automatic regulation of cuff pressure,
   ∘ a low cost and easy to use device for automatic regulation of cuff pressure,
   ∘ an adjustable device for different cuff pressure ranges.

## Claims

1. A device (100) for regulating cuff pressure in endotracheal tubes, comprising
a barrel (10) for guiding a plunger (20),
the plunger (20) being adapted to travel along a main longitudinal axis (A) of said barrel, thereby defining a cavity (C) with variable volume within the barrel,
the device (100) further comprises a passage (11) adapted to allow fluid transfer communication to and from the cavity (C), and
one or more biasing member (30) adapted to bias the plunger (20) towards said passage (11), **wherein**
the passage (11) is provided with a tee-connection (50) comprising a first conduit (51), a second conduit (52) and a third conduit (53) connected together at one end of each conduit;
- the first conduit (51) comprises a first check valve (511) adapted to allow one-way fluid transfer towards the cavity (C),
- the second conduit (52) comprises a second check valve (512) adapted to allow one-way fluid transfer from the cavity (C), and
- the third conduit (53) is adapted to provide fluid transfer communication between the cavity (C) and both of the first conduit (51) and the second conduit (52).

2. Device according to the claim 1, further comprising one or more visual indicator (40) adapted to allow visual monitoring of a magnitude related to the relative position of the plunger (20) with respect to the passage (11).

3. Device according to any of the claims 1 or 2, wherein the plunger and the barrel are arranged to define a limited range of motion to the plunger, said range of motion being in accordance with a first value of force (F1) and a second value of force (F1) exerted onto the plunger (20) by the biasing member (30).

4. Device according to any of the claims 2 or 3, wherein the visual indicator (40) comprises a graduated marking scale (40') at a projection mainly parallel to said axis (A), in combination with a position indicating member (22) secured to the plunger (20), adapted to enable visual monitoring of the position (P) of the position indicating member (22) relative to said scale (40');
the scale (40') being provided with at least a first marker (41) and a second marker (42) which is at a distance different than that of the first marker (41) relative to the passage (11).

5. Device according to ant of the claims 1 to 4, provided in a sleeve at least partly covering the device (100), the sleeve being adapted to allow manual positioning of the plunger inside the barrel, and adapted to allow visual monitoring of the visual indicator from outside of the sleeve.

6. Device according to the claim 5, wherein the sleeve is provided with one or more slit adapted to allow the position indicating member to protrude through.

7. Device according to the claim 6, wherein the sleeve is mainly opaque and the scale is provided on an outer surface of the sleeve.

8. Device according to any of the claims 1 to 7, wherein the biasing member (30) comprises a resilient member which comprises one or more compression spring adapted to push the plunger (20) towards the passage (11).

9. Device according to any of the claims 1 to 7, wherein the biasing member (30) comprises a resilient member which comprises one or more tension spring and/or one or more elongate elastic polymeric member adapted to pull the plunger (20) towards the passage (11).

10. Device according to any of the claims 1 to 7, wherein the biasing member (30) comprises a resilient member which comprises one or more compression spring adapted to push the plunger (20) towards the passage (11); and a further resilient member which comprises one or more tension spring and/or one or more elongate elastic polymeric member adapted to pull the plunger (20) towards the passage (11).

11. Device according to any of the claims 3 to 10, wherein the biasing member (30) and the scale (40') are arranged so that a maximum pressure value acceptable inside a cuff is indicated by the first marker (41) corresponding to the first amount of force to be exerted by the biasing member (30) in case where a projection on the pressure indicating member (22) mainly perpendicular to the axis (A) coincides with the first marker (41), and a minimum pressure value acceptable inside a cuff is indicated by the second marker (42) corresponding to the second amount of force to be exerted by the biasing member (30) in case where the projection of the pressure indicating member (22) mainly perpendicular to said axis (A) coincides with the second marker (42).

12. A kit including an endotracheal tube with a cuff and the device (100) according to any of the claims 1 to 11 adapted to couple with the cuff.

13. An endotracheal tube comprising a cuff and the device (100) according to any of the claims 1 to 11 adapted to couple with the cuff.

## Patentansprüche

1. Vorrichtung (100) zum Regulieren des Manschettendrucks in Endotrachealtuben, aufweisend einen Zylinder (10) zum Führen eines Kolbens (20),
wobei der Kolben (20) so angepasst ist, dass er sich entlang einer Hauptlängsachse (A) des Zylinders bewegt, wodurch ein Hohlraum (C) mit variablem Volumen innerhalb des Zylinders definiert wird,
wobei die Vorrichtung (100) ferner einen Durchgang (11) aufweist, der so angepasst ist, dass er eine Fluidtransferverbindung zu und von dem Hohlraum (C) ermöglicht,
und ein oder mehrere Vorspannelement(e) (30), das bzw. die geeignet ist bzw. sind, den Kolben (20) in Richtung des Durchgangs (11) vorzuspannen, **wobei**
der Durchgang (11) mit einem T-Verbindungsstück (50) versehen ist, das eine erste Leitung (51), eine zweite Leitung (52) und eine dritte Leitung (53) aufweist, die an einem Ende jeder Leitung miteinander verbunden sind;
- die erste Leitung (51) ein erstes Rückschlagventil (511) aufweist, das so angepasst ist, dass es einen Einweg-Fluidtransfer in Richtung des Hohlraums (C) ermöglicht,
- die zweite Leitung (52) ein zweites Rückschlagventil (512) aufweist, das so angepasst ist, dass es einen Einweg-Fluidtransfer aus dem Hohlraum (C) ermöglicht, und
- die dritte Leitung (53) so angepasst ist, dass sie eine Fluidtransferverbindung zwischen dem Hohlraum (C) und sowohl der ersten Leitung (51) als auch der zweiten Leitung (52) bereitstellt.

2. Vorrichtung nach Anspruch 1, die weiterhin eine oder mehrere visuelle Anzeige(n) (40) aufweist, die geeignet ist/sind, eine visuelle Überwachung einer Größe zu ermöglichen, die mit der relativen Position des Kolbens (20) in Bezug auf den Durchgang (11) in Beziehung steht.

3. Vorrichtung nach einem der Ansprüche 1 oder 2, wobei der Kolben und der Zylinder so angeordnet sind, dass sie einen begrenzten Bewegungsbereich des Kolbens definieren, wobei der Bewegungsbereich einem ersten Kraftwert (F1) und einem zweiten Kraftwert (F1) entspricht, die durch das Vorspannelement (30) auf den Kolben (20) ausgeübt werden.

4. Vorrichtung nach einem der Ansprüche 2 oder 3, wobei die visuelle Anzeige (40) eine abgestufte Markierungsskala (40') in einer im Wesentlichen parallel zur Achse (A) verlaufenden Projektion in Kombination mit einem am Kolben (20) befestigten Positionsanzeigeelement (22) aufweist, die geeignet ist, die visuelle Überwachung der Position (P) des Positionsanzeigeelements (22) relativ zur Skala (40') zu ermöglichen;
wobei die Skala (40') mit mindestens einer ersten Markierung (41) und einer zweiten Markierung (42) versehen ist, die sich in einem anderen Abstand als die erste Markierung (41) relativ zu dem Durchgang (11) befindet.

5. Vorrichtung nach den Ansprüchen 1 bis 4, die in einer Hülle vorgesehen ist, die die Vorrichtung (100) zumindest teilweise bedeckt, wobei die Hülle dazu geeignet ist, eine manuelle Positionierung des Kolbens im Inneren des Zylinders zu ermöglichen, und dazu geeignet ist, eine visuelle Überwachung der visuellen Anzeige von außerhalb der Hülle zu ermöglichen.

6. Vorrichtung nach Anspruch 5, wobei die Hülle mit einem oder mehreren Schlitz(en) versehen ist, der (die) so ausgebildet ist (sind), dass das Positionsanzeigeelement durch sie hindurchragen kann (können).

7. Vorrichtung nach Anspruch 6, wobei die Hülle hauptsächlich undurchsichtig ist und die Skala auf einer äußeren Oberfläche der Hülle vorgesehen ist.

8. Vorrichtung nach einem der Ansprüche 1 bis 7, wobei das Vorspannelement (30) ein elastisches Element aufweist, das eine oder mehrere Druckfedern aufweist, die geeignet sind, den Kolben (20) in Richtung des Durchgangs (11) zu drücken.

9. Vorrichtung nach einem der Ansprüche 1 bis 7, wobei das Vorspannelement (30) ein elastisches Element aufweist, das eine oder mehrere Zugfedern und/oder ein oder mehrere längliche elastische Polymerelemente aufweist, die geeignet sind, den Kolben (20) in Richtung des Durchgangs (11) zu ziehen.

10. Vorrichtung nach einem der Ansprüche 1 bis 7, wobei das Vorspannelement (30) ein elastisches Element aufweist, das eine oder mehrere Druckfedern aufweist, die geeignet sind, den Kolben (20) in Richtung des Durchgangs (11) zu drücken; und ein weiteres elastisches Element, das eine oder mehrere Zugfedern und/oder ein oder mehrere längliche elastische Polymerelemente aufweist, die geeignet sind, den Kolben (20) in Richtung des Durchgangs (11) zu ziehen.

11. Vorrichtung nach einem der Ansprüche 3 bis 10, wobei das Vorspannelement (30) und die Skala (40') so angeordnet sind, dass ein maximaler Druckwert, der innerhalb einer Manschette akzeptabel ist, durch die erste Markierung (41) angezeigt wird, die dem ersten Kraftbetrag entspricht, der durch das Vorspannelement (30) in dem Fall ausgeübt wird, dass eine Projektion auf dem Druckanzeigeelement (22), die hauptsächlich senkrecht zur Achse (A) ist, mit der ersten Markierung (41) zusammenfällt, und ein minimaler Druckwert, der innerhalb einer Manschette akzeptabel ist, durch die zweite Markierung (42) angezeigt wird, die dem zweiten Kraftbetrag entspricht, der durch das Vorspannelement (30) in dem Fall auszuüben ist, dass die Projektion des Druckanzeigeelements (22), die hauptsächlich senkrecht zu der Achse (A) ist, mit der zweiten Markierung (42) zusammenfällt.

12. Kit, das einen Endotrachealtubus mit einer Manschette und die Vorrichtung (100) nach einem der Ansprüche 1 bis 11 enthält, die zur Kopplung mit der Manschette geeignet ist.

13. Endotrachealtubus mit einer Manschette und der Vorrichtung (100) nach einem der Ansprüche 1 bis 11, die zur Kopplung mit der Manschette geeignet ist.

## Revendications

1. Dispositif (100) de régulation de pression de brassard dans des tubes endotrachéaux, comprenant
un cylindre (10) pour guider un piston (20),
le piston (20) étant adapté pour se déplacer le long d'un axe longitudinal principal (A) dudit cylindre, définissant ainsi une cavité (C) ayant un volume variable à l'intérieur du cylindre,
le dispositif (100) comprend en outre un passage (11) adapté pour permettre une communication de transfert de fluide vers et depuis la cavité (C), et
un ou plusieurs éléments de sollicitation (30) adaptés pour solliciter le piston (20) vers ledit passage (11),
le passage (11) étant muni d'un raccordement en T (50) comprenant un premier conduit (51), un deuxième conduit (52) et un troisième conduit (53) reliés ensemble à une extrémité de chaque conduit ;
- le premier conduit (51) comprend un premier clapet anti-retour (511) adapté pour permettre un transfert de fluide unidirectionnel vers la cavité (C),
- le deuxième conduit (52) comprend un second clapet anti-retour (512) adapté pour permettre un transfert de fluide unidirectionnel depuis la cavité (C), et
- le troisième conduit (53) est adapté pour assurer une communication de transfert de fluide entre la cavité (C) et à la fois le premier conduit (51) et le deuxième conduit (52).

2. Dispositif selon la revendication 1, comprenant en outre un ou plusieurs indicateurs visuels (40) adaptés pour permettre une surveillance visuelle d'une grandeur liée à la position relative du piston (20) par rapport au passage (11).

3. Dispositif selon l'une quelconque des revendications 1 ou 2, dans lequel le piston et le cylindre sont disposés de manière à définir une amplitude de mouvement limitée pour le piston, ladite amplitude de mouvement étant conforme à une première valeur de force (F1) et à une seconde valeur de force (F1) exercées sur le piston (20) par l'élément de sollicitation (30).

4. Dispositif selon l'une quelconque des revendications 2 ou 3, dans lequel l'indicateur visuel (40) comprend une échelle de repérage graduée (40') à une projection principalement parallèle audit axe (A), en combinaison avec un élément d'indication de position (22) fixé au piston (20), adapté pour permettre une surveillance visuelle de la position (P) de l'élément d'indication de position (22) par rapport à ladite échelle (40') ;
l'échelle (40') étant munie au moins d'un premier repère (41) et d'un second repère (42) qui est à une distance différente de celle du premier repère (41) par rapport au passage (11).

5. Dispositif selon l'une quelconque des revendications 1 à 4, disposé dans un manchon couvrant au moins partiellement le dispositif (100), le manchon étant adapté pour permettre un positionnement manuel du piston à l'intérieur du cylindre, et adapté pour permettre une surveillance visuelle de l'indicateur visuel depuis l'extérieur du manchon.

6. Dispositif selon la revendication 5, dans lequel le manchon est muni d'une ou plusieurs fentes adaptées pour permettre à l'élément d'indication de position de faire saillie à travers celles-ci.

7. Dispositif selon la revendication 6, dans lequel le manchon est principalement opaque et l'échelle est disposée sur une surface externe du manchon.

8. Dispositif selon l'une quelconque des revendications 1 à 7, dans lequel l'élément de sollicitation (30) comprend un élément élastique qui comprend un ou plusieurs ressorts de compression adaptés pour pousser le piston (20) vers le passage (11).

9. Dispositif selon l'une quelconque des revendications 1 à 7, dans lequel l'élément de sollicitation (30) comprend un élément élastique qui comprend un ou plusieurs ressorts de tension et/ou un ou plusieurs éléments polymères élastiques allongés adaptés pour tirer le piston (20) vers le passage (11).

10. Dispositif selon l'une quelconque des revendications 1 à 7, dans lequel l'élément de sollicitation (30) comprend un élément élastique qui comprend un ou plusieurs ressorts de compression adaptés pour pousser le piston (20) vers le passage (11) ; et un élément élastique supplémentaire qui comprend un ou plusieurs ressorts de tension et/ou un ou plusieurs éléments polymères élastiques allongés adaptés pour tirer le piston (20) vers le passage (11).

11. Dispositif selon l'une quelconque des revendications 3 à 10, dans lequel l'élément de sollicitation (30) et l'échelle (40') sont disposés de telle sorte qu'une valeur de pression maximale acceptable à l'intérieur d'un brassard est indiquée par le premier repère (41) correspondant à la première quantité de force à exercer par l'élément de sollicitation (30) dans le cas où une projection sur l'élément d'indication de pression (22) principalement perpendiculaire à l'axe (A) coïncide avec le premier repère (41), et une valeur de pression minimale acceptable à l'intérieur d'un brassard est indiquée par le second repère (42) correspondant à la seconde quantité de force à exercer par l'élément de sollicitation (30) dans le cas où la projection de l'élément d'indication de pression (22) principalement perpendiculaire audit axe (A) coïncide avec le second repère (42).

12. Kit comprenant un tube endotrachéal avec un brassard et le dispositif (100) selon l'une quelconque des revendications 1 à 11 adapté pour se coupler au brassard.

13. Tube endotrachéal comprenant un brassard et le dispositif (100) selon l'une quelconque des revendications 1 à 11 adapté pour se coupler au brassard.
